# NOUVEAU FASCICULE DE BREVET EUROPEEN

(11) **EP 0 684 306 B2**
(45) Date de publication et mention de la décision concernant l'opposition: **14.01.2004**
(45) Mention de la délivrance du brevet: 15.12.1999
(21) Numéro de dépôt: 94810307.2
(22) Date de dépôt: 27.05.1994
(51) Int. Cl.: C12N 1/18, A21D 8/04, C12N 1/20, C12M 1/36, C12C 7/00, C12P 7/06

(54) **Procédé d'obtention d'une biomasse sur un milieu céréalier, utilisation des produits résultant de ce procédé et ferment de panification**
Verfahren zur Gewinnung einer Biomasse aus einem Medium von Getreide, Verwendung der erhaltenen Produkte und Brottreibmittel
Process for production of biomass from a cereal medium, use of the obtained products and bread leavening agent

(43) Date de publication de la demande: 29.11.1995
(73) Titulaire: AGRANO AG, CH-4123 Allschwil (CH)
(72) Inventeur: Ehret,Aloyse, F-68730 Blotzheim (FR)
(74) Mandataire: Rottmann, Maximilian R.

(56) Documents cités:
- EP-A- 0 093 635
- EP-A- 0 229 979
- EP-A- 0 295 358
- WO-A-91/04669
- WO-A-92/20777
- BE-A- 816 571
- FR-A- 2 353 235
- GB-A- 372 738
- GB-A- 1 035 552
- US-A- 3 868 307
- DATABASE WPI Section Ch, Week 8822, Derwent Publications Ltd., London, GB; Class D16, AN 88-153243 & SU-A-1 346 676 (KAUN POLY) 23 Octobre 1987
- J.ADRIAN, G. LEGRAND, R. FRANGNE: "Dictionnaire de Biochimie Alimentaire et de Nutrition p.79" , 1981, TECHNIQUE ET DOCUMENTATION, PARIS

## Description

L'invention a trait
- à un procédé d'obtention d'une biomasse sur un milieu céréalier, tel que défini dans les revendications 1 à 10;
- à l'utilisation du produit obtenu comme ferment de panification, telle que définie dans la revendication 11;
- aux utilisations d'eaux-mères résultant du procédé, telles que définies dans les revendications 12 et 13.

Actuellement la levure utilisée en panification est obtenue industriellement à partir de cultures faites dans des milieux à base de mélasses de betteraves ou de canne à sucre, additionnés de suppléments vitaminiques et de composés chimiques, tels que l'acide phosphorique et l'ammoniaque. Cette levure, récoltée par centrifugation et par filtration, permet une panification rapide mais ne confère pas aux produits finis résultant de la mise en oeuvre les qualités organoleptiques que l'on trouve dans un produit de panification artisanale obtenu à partir d'un levain.

De plus, la publication EP-A1-0.093.635 décrit une composition fermentée pour la préparation d'un levain de panification et un procédé permettant la préparation d'un tel levain par culture de souches sur un milieu céréalier. Le milieu utilisé comprend de l'eau, de la farine, du germe de blé, de l'extrait de levure, du chlorure d'ammonium, du dihydrogénate de potassium, de l'antimousse, de l'alpha amylase et de l'amyloglucosidase. Le procédé de production n'envisage cependant pas l'utilisation de protéases. De plus, la réaction amyloglycolytique est effectué à pH 4.5, ce qui nécessite l'ajout d'acide et donc un contrôle du pH. La composition ainsi que le procédé de sa production ne sont donc pas "tout biologique".

Finalement, la publication WO-A1-92/20.777 décrit un procédé visant à produire de l'éthanol à partir de matières premières contenant des sucres fermentescibles ou des constituants pouvant être convertis en sucres fermentescibles. Le dit procédé comprend les étapes suivantes: (a) liquéfaction des matières premières en présence d'une α-amylase afin d'obtenir une liquéfaction de l'amidon (empois); (b) saccharification de l'empois en présence de glucoamylase pour obtenir les sucres fermentescibles; (c) fermentation des sucres par la levure afin d'obtenir de l'éthanol; et (d) séparation de l'éthanol formé, une protéase fongique étant introduite dans l'empois d'amidon pendant la phase de saccharification et/ou à l'amidon hydrolysé durant la fermentation.

Le but du procédé de cette référence n'est donc pas de produire un milieu de culture qui peut être utilisé pour la culture individuelle de levure et de bactéries lactiques ainsi que pour la coculture de levure et de bactéries lactiques mais la production d'éthanol.

Or, le but de la présente invention est de remédier à ces inconvénients en créant un procédé d'obtention d'une biomasse, sur un milieu céréalier, le produit étant directement utilisable comme ferment de panification sans séparation préalable entre milieu de culture et biomasse et sans ajout de levure industrielle.

Ce but est atteint par les mesures définies dans la partie caractérisante de la revendication 1.

L'exécution de la culture dans le milieu de culture décrit ci-dessus garantit que les produits de panification obtenus présentent des qualités organoleptiques particulières reconnues.

La culture est préférablement du type discontinu alimenté, dit "en fed-batch". Cette technique consiste à additionner une charge de milieu périodiquement en continu avec les ingrédients nécessaires. Ce type de fermentation permet de contrôler le métabolisme de la culture entre voie aérobie et voie anaérobie, contrôlant ainsi les flux métaboliques entre croissance (biomasse) et les produits dérivés de son métabolisme (en particulier éthanol).

La culture se fait sans régulation de pH, évitant ainsi l'adjonction d'additifs chimiques.

De préférence, on contrôle la teneur en éthanol dans la culture par régulation de la vitesse d'alimentation en milieu de culture.

La pression partielle de l'oxygène dissout dans la culture peut être contrôlée par régulation de l'apport d'air et/ou par régulation de la vitesse d'agitation, selon une pente préalablement définie, tout en maintenant la culture dans un métabolisme limité en oxygène.

De préférence, la levure mise en oeuvre est une souche de *Saccharomyces cerevisiae,* préférablement une souche isolée d'un levain naturel, en particulier la souche *Saccharomyces cerevisiae* steineri DSM 9211. Cette souche a été isolée à partir d'un levain artisanal d'excellente qualité organoleptique. Les caractères du genre *Saccharomyces cerevisiae* sont compilés dans la Tableau 1.

### Tableau 1

### Caractéristiques de Saccharomyces cerevisiae

Culture en milieu liquide (milieu de dosage dilué au ½):
Observation au microscope: Les cellules de levure sont ovales (2 à 4 x 5 à 7 micromètres) et se divisent par bourgeonnement multipolaire.

Culture en milieu solide (milieu de dosage dilué au ½ additionné de 15 gramme/litre de gélose):
Observation des colonies: Les colonies de levure sont rondes, lisses, mates, légèrement bombées et de couleur crème.

### Métabolisme des sucres:

| | Assimilation | Fermentation |
|---|---|---|
| Glucose | + | + |
| L-Arabinose | - | - |
| D-Xylose | - | - |
| Galactose | + | + |
| Cellobiose | - | - |
| Lactose | - | - |
| Maltose | + | + |
| Saccharose | + | + |
| Tréhalose | - | - |
| Mélézitose | - | - |
| Raffinose | + | - |

La mise en oeuvre du procédé selon l'invention se fait préférablement dans un bioréacteur en procédant comme ceci:
- on introduit du milieu de culture dans un bioréacteur;
- on ensemence ce milieu de culture avec de la levure;
- on alimente le bioréacteur en continu avec du milieu de culture;
tout en maintenant une température d'environ 30 °C, une aération contrôlée par la pression partielle d'oxygène, et une concentration en éthanol entre 0,5 et 10 gramme/litre de culture.

Le produit obtenu peut être utilisé, dès sa sortie du bioréacteur, soit comme ferment en panification directe industrielle sans opération ultérieure, soit sans séparation préalable entre milieu de culture et biomasse. Cependant, il est en règle générale préférable de concentrer le produit par centrifugation ou par filtration, puisqu'alors le produit réduit en sa teneur d'eau peut être conservé à moins de 3°C pendant 21 jours sans perte notable de son pouvoir levant.

Aussi, dans ce cas, les eaux-mères résultant de la concentration peuvent être utilisées pour la fabrication d'un substrat de culture de bactéries lactiques ou pour la fabrication d'une boisson.

### Exemple

Dans un bioréacteur de 15 litres (volume utile 10 litres) on introduit 5 litres d'un milieu de culture, dit "milieu de base", dont la composition est la suivante:
- 5 litres d'eau;
- une source d'azote, par exemple 250 grammes de germe de blé;
- une source de vitamines, d'acides aminés et de sels minéraux, par exemple 35 grammes d'autolysat de levure; et
- 15 grammes de sel marin.

A ce milieu de base est ajoutée une souche de levure identifiée à l'espèce *Saccharomyces cerevisiae steineri,* déposée à la Collection allemande de microorganismes (DMS) sous le N° 9211. Cette souche a été cultivée sur un milieu, dit "milieu de dosage", dont la composition est la suivante:
- 4 litres d'eau;
- une source de carbone, par exemple 800 grammes de grains de blé moulus;
- une source d'azote, par exemple 500 grammes de germe de blé;
- une source de vitamines, d'acides aminés et de sels minéraux, par exemple 70 grammes d'autolysat de levure; et
- 15 grammes de sel marin.

La souche est conservée à -80 °C dans le milieu céréalier (milieu de dosage dilué au ½ avec de l'eau). La souche est réisolée sur le milieu céréalier solide, et elle est entretenue sur le même milieu par repiquages successifs tous les 15 jours.

Une colonie isolée est ensemencée dans le milieu céréalier liquide. Une deuxième culture est faite avec 20 ml de la première dans un Erlenmeyer de 500 ml contenant 200 ml de milieu céréalier. La densité cellulaire obtenue après 16 heures de culture avec agitation est de 3.10⁸ cellules par ml. Une troisième culture à partir de la deuxième est faite dans 600 ml de la culture précédente. La densité cellulaire obtenue après 8 heures à 30 °C est de 2,5.10⁸ cellules par ml. Le glucose est entièrement métabolisé, et le teneur en éthanol mesurée est voisine de 25 gramme/litre.

600 ml de cette culture de levure en phase exponentielle sont ajoutés aux 5 litres du milieu de base. Le mélange est alimenté en continu en milieu de dosage. La température est maintenue à 30 °C, le pH mesuré en permanence n'est pas régulé, et la pO₂ est maintenue supérieure à 30 %.

La vitesse d'alimentation est asservie à deux paramètres essentiels au bon déroulement du procédé:

### (a) Le paramètre concentration en éthanol du milieu de culture:

La levure est un microorganisme capable de se multiplier en aérobiose et/ou en anaérobiose. Le métabolisme aérobie favorise l'accroissement de la biomasse, alors que le métabolisme anaérobie conduit à la production d'éthanol et de dérivés secondaires recherchés pour leurs qualités organoleptiques. Le procédé selon l'invention permet de contrôler les flux métaboliques entre ces deux voies extrêmes, permettant une bonne croissance et par là l'obtention d'une biomasse finale capable d'être utilisée en panification directe dans des conditions de temps dites "industrielles".

La production d'éthanol traduit un flux métabolique dans la voie anaérobie.

Dans le procédé décrit, la concentration en éthanol est systématiquement recherchée et maintenue entre 0,5 et 10 gramme/litre. Si la concentration passe sous le seuil de 0,5 gramme/litre, la vitesse d'alimentation est augmentée en milieu de dosage. L'alimentation est ralentie en cas de dépassement du seuil de 10 gramme/litre.

### (b) L'aération du milieu de culture:

Ce paramètre est déterminé en continu par la mesure de l'oxygène dissout et est régulé de deux façons, à savoir par pilotage de l'entrée d'air dans le bioréacteur et/ou par l'asservissement de la vitesse de rotation de deux bipales de type Rushton (taille ½ du diamètre du bioréacteur) permettant de piloter le transfert d'O₂.

Dans le procédé décrit, la pression partielle d'O₂ est maintenue supérieure à 10 %. La vitesse d'agitation varie de 500 à 1200 rpm, et le débit d'air stérile passe de 0 à 30 litres/minute.

Dans les conditions décrites, la culture est alimentée durant 16 heures. L'alimentation totale est de 4 litres durant cette période. La densité cellulaire de levure atteint 2.10⁹ cellules par ml (densité de départ 2,5.10⁷ cellules par ml).

La culture obtenue est rapidement refroidie à 3 °C et peut ensuite être conservée sans pertes notables de ses qualités durant 21 jours.

### Variante:

Une postfermentation sur farine à raison de 300 gramme/litre de ferment pendant 6 heures à 20 °C suivie d'un refroidissement à 3 °C permet une conservation pendant 30 jours et l'obtention d'un produit de panification aux qualités organoleptiques de type "levain".

### Panification:

Les ferments liquides (issus directement du bioréacteur ou après postfermentation) sont utilisés directement à une concentration de 20 % (poids de farine/volume de ferment) pour ensemencer une pâte traditionnelle faite de farine, d'eau et de sel.

### Variante:

Les ferments sont centrifugés. La réduction de la teneur en eau augmente leur stabilité et permet de diminuer sensiblement la concentration dans le procédé de panification. Ainsi une centrifugation à 8500 G permet d'obtenir un ferment à 75 % d'eau et pourra être utilisé à une concentration de 6 à 8 % dans le mélange de panification.

La densité des pains obtenus est identique à celle obtenue avec la levure pressée traditionnelle (cultivée sur mélasse sucrière) et utilisée à une concentration de 2 à 3 %.

L'analyse de l'exemple donné ci-dessus est compilé dans le Tableau 2.

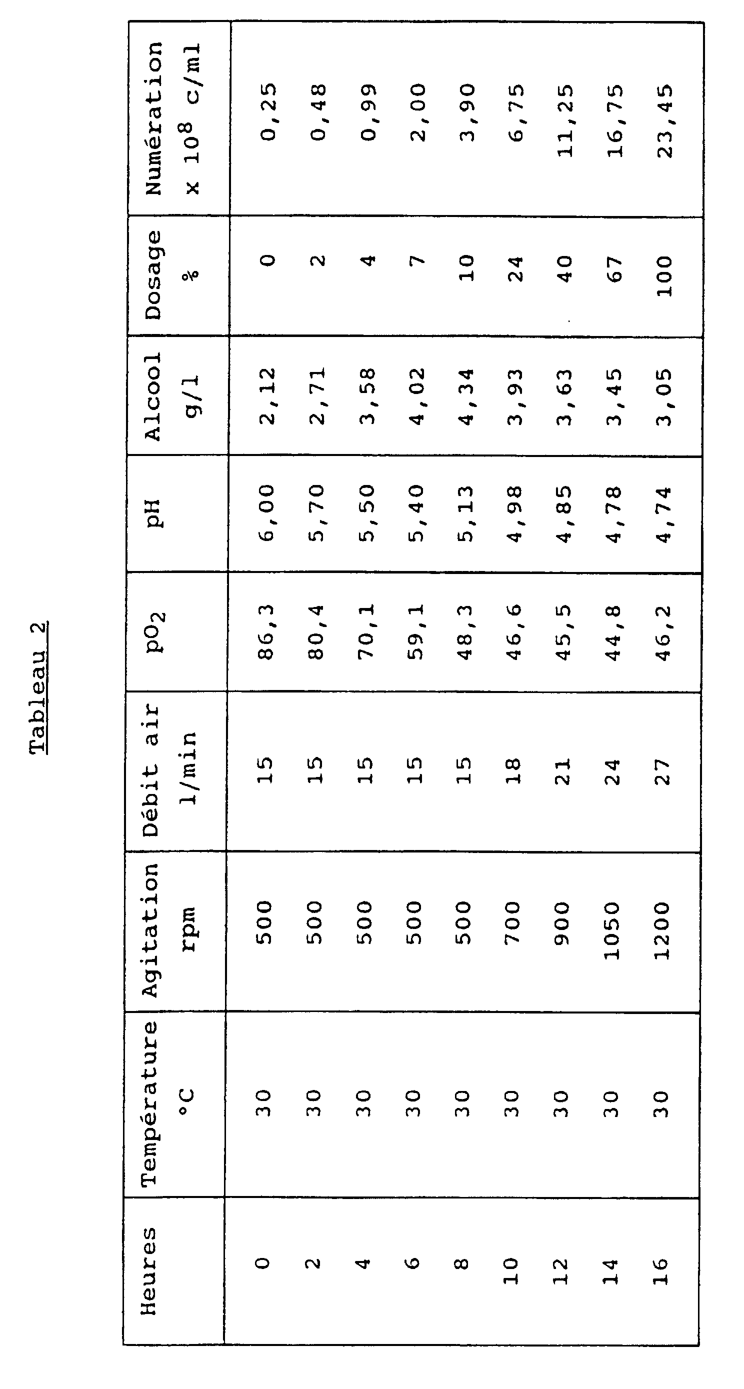

## Revendications

1. Procédé d'obtention d'une biomasse, sur un milieu céréalier, le produit étant directement utilisable comme ferment de panification sans séparation préalable entre milieu de culture et biomasse et sans ajout de levure industrielle, **caractérisé en ce qu'**on cultive sans régulation de pH au moins une souche de levure sur un milieu de culture obtenu:
- par une double hydrolyse d'un mélange dilué aqueux consistant en de l'eau, de la farine intégrale et/ou du germe de blé ainsi que de l'autolysat de levure et du sel marin, à savoir
- par l'hydrolyse totale de l'amidon en sucres fermentescibles par l'action d'au moins une alpha-amylase et d'au moins une amyloglucosidase, et
- par l'hydrolyse avec ménagement d'au moins une partie du gluten par des enzymes protéolytiques de qualité alimentaire;
et
- ce milieu de culture étant exempt de tout additif chimique autre que les ingrédients mentionnés ci-dessus.

2. Procédé selon la revendication 1, carectérisé en ce que la culture est du type discontinu alimenté.

3. Procédé selon la revendication 1 ou 2, **caractérisé en ce qu'**on contrôle la teneur en éthanol dans la culture par régulation de la vitesse d'alimentation en milieu de culture.

4. Procédé selon une des revendications 1 à 3, **caractérisé en ce qu'**on contrôle la pression partielle de l'oxygène dissout dans la culture par régulation de l'apport d'air suivant une pente préalablement définie maintenant la culture dans un métabolisme limité en oxygène.

5. Procédé selon une des revendications 1 à 4, **caractérisé en ce qu'**on contrôle la pression partielle de l'oxygène dissout dans la culture par la vitesse d'agitation suivant une pente préalablement définie maintenant la culture dans un métabolisme limité en oxygène.

6. Procédé selon une des revendications 1 à 5, **caractérisé en ce qu'**on
- introduit un milieu de base dans un bioréacteur,
- ensemence ce milieu de base avec de la levure distribuée dans un milieu de dosage;
- alimente le bioréacteur en continu avec le milieu de dosage;
tout en maintenant une température d'environ 30 °C, une aération contrôlée par la pression partielle d'oxygène, et une concentration en éthanol entre 0,5 et 10 gramme/litre de culture.

7. Procédé selon une des revendications 1 à 6, **caractérisé en ce qu'**on concentre le produit obtenu par centrifugation ou par filtration.

8. Procédé selon une des revendications 1 à 7, **caractérisé en ce que** la levure mise en oeuvre est une souche de *Saccharomyces cerevisiae.*

9. Procédé selon la revendication B, **caractérisé en ce que** la levure mise en oeuvre est une souche de *Saccharomyces cerevisiae* isolée d'un levain naturel.

10. Procédé selon la revendication 9, **caractérisé en ce que** la levure mise en oeuvre est la souche *Saccharomyces cerevisiae steineri* DSM 9211.

11. Utilisation du produit sans séparation préalable entre milieu de culture et biomasse obtenu selon une des revendications 1 à 6 comme ferment de panification.

12. Utilisation des eaux-mères résultant de la concentration selon la revendication 7 pour la fabrication d'un substrat de culture de bactéries lactiques.

13. Utilisation des eaux-mères résultant de la concentration selon la revendication 7 pour la fabrication d'une boisson.

## Patentansprüche

1. Verfahren zur Gewinnung einer Biomasse auf einem Getreidemedium, wobei das erhaltene Produkt ohne vorgängige Trennung von Kulturmedium und Biomasse und ohne Zusatz von Industrieller Hefe direkt als Ferment für die Brotherstellung verwendbar ist, **dadurch gekennzeichnet, dass** man ohne pH-Regulierung mindestens einen Hefestamm auf einem Medium züchtet, welches erhalten wurde:
- durch eine doppelte Hydrolyse einer verdünnten wässerigen Mischung, welche aus Wasser, Vollkornmehl und/oder Weizenkeimen sowie Hefeautolysat und Meersalz besteht, nämlich
- durch die totale Hydrolyse der Stärke zu vergärbaren Zuckern durch Einwirkung wenigstens einer alpha-Amylase und wenigstens einer Amyloglucosidase, und
- durch gesteuerte Hydrolyse wenigstens eines Teils des Glutens durch proteolytische Enzyme von Lebensmittelqualität;
wobei dieses Kulturmedium frei ist von jeglichen chemischen Zusätzen ausser den vorstehend genannten.

2. Verfahren nach Anspruch 2, **dadurch gekennzeichnet, dass** die Kultur vom Typ versorgte diskontinuierliche Kultur ist.

3. Verfahren nach Anspruch 1 oder 2, **dadurch gekennzeichnet, dass** man den Äthanolgehalt in der Kultur durch Regulierung der Versorgungsgeschwindigkeit des Kulturmediums steuert.

4. Verfahren nach einem der Ansprüche 1 bis 3, **dadurch gekennzeichnet, dass** man den Partialdruck des in der Kultur gelösten Sauerstoffs durch Regulierung der Luftzufuhr gemäss einer vorbestimmten Steigung steuert, wobei die Kultur bei einem beschränkten Sauerstoffstoffwechsel gehalten wird.

5. Verfahren nach einem der Ansprüche 1 bis 3, **dadurch gekennzeichnet, dass** man den Partialdruck des in der Kultur gelösten Sauerstoffs durch Regulierung der Rührgeschwindigkeit gemäss einer vorbestimmten Steigung steuert, wobei die Kultur bei einem beschränkten Sauerstoffstoffwechsel gehalten wird.

6. Verfahren nach einem der Ansprüche 1 bis 5, **dadurch gekennzeichnet, dass** man:
- ein Basismilieu in einen Bioreaktor einführt;
- dieses Basismilieu mit der zu kultivierenden Hefe, verteilt in einem Dosiermilieu, beimpft; und
- den Bioreaktor kontinuierlich mit dem Dosiermedium versorgt,
wobei eine Temperatur von etwa 30 °C, über den Sauerstoffpartialdruck eine gesteuerte Luftzufuhr sowie eine Äthanolkonzentration zwischen 0,5 und 10 Gramm/Liter aufrechterhalten wird.

7. Verfahren nach einem der Ansprüche 1 bis 6, **dadurch gekennzeichnet, dass** man das erhaltene Produkt durch Zentrifugation oder Filtration konzentriert.

8. Verfahren nach einem der Ansprüche 1 bis 7, **dadurch gekennzeichnet, dass** die eingesetzte Hefe ein Stamm von *Saccharomyces cerevisiae* ist.

9. Verfahren nach Anspruch 8, **dadurch gekennzeichnet, dass** die eingesetzte Hefe ein aus einem natürlichen Hebel isolierter Stamm von *Saccharomyces cerevisiae* ist.

10. Verfahren nach Anspruch 9, **dadurch gekennzeichnet, dass** die eingesetzte Hefe der Stamm *Saccharomyces cerevisiae steineri* DSM 9211 ist.

11. Verwendung des nach einem der Ansprüche 1 bis 10 erhaltenen Produktes ohne vorherige Trennung von Kulturmilieu und Biomasse als Ferment für die Brotherstellung.

12. Verwendung der Mutterlaugen der Konzentrierung nach Anspruch 7 zur Herstellung eines Kultursubstrates für Milchsäurebakterien.

13. Verwendung der Mutterlaugen der Konzentrierung nach Anspruch 7 zur Herstellung eines Getränks.

## Claims

1. Method of producing a biomass, on a cereal medium, the product being directly utilizable as ferment for breadmaking without prior separation between the culture medium and biomass and without addition of industrial yeast, **characterized in that** at least one yeast strain is cultured, without regulation of pH, on a culture medium obtained:
- by a double hydrolysis of an aqueous dilute mixture consisting of water, wholemeal flour and/or wheat germ as well as yeast autolysate and sea salt, namely
- by the complete hydrolysis of starch to fermentable sugars by the action of at least one alpha-amylase and at least one amyloglucosidase, and
- by the controlled hydrolysis of at least part of the gluten by food-grade proteolytic enzymes;
- this culture medium being free of any chemical additive other than the ingredients mentioned above.

2. Method according to Claim 1, **characterized in that** the culture is of the fed-batch type.

3. Method according to Claim 1 or 2, **characterized in that** the content of ethanol in the culture is controlled by regulating the rate of feeding the culture medium.

4. Method to one of Claims 1 to 3, **characterized in that** the partial pressure of oxygen dissolved in the culture is controlled by regulating the supply of air according to a previously defined slope maintaining the culture in an oxygen-limited metabolism.

5. Method according to one of Claims 1 to 4, **characterized in that** the partial pressure of oxygen dissolved in the culture is controlled by the rate of stirring according to a previously defined slope maintaining the culture in an oxygen-limeted metabolism.

6. Method according to one of Claims 1 to 5, **characterized in that**
- a basal medium is introduced into a bioreactor;
- this basal medium is inoculated with yeast distributed in a proportioning medium;
- the bioreactor is continuously fed with the proportioning medium;
while maintaining a temperature of about 30°C, a controlled aeration by the partial pressure of oxygen and an ethanol concentration of between 0.5 and 10 grams/litre of culture.

7. Method according to one of Claims 1 to 6, **characterized in that** the product obtained is concentrated by centrifugation or filtration.

8. Method according to one of Claims 1 to 7, **characterized in that** the yeast used is a strain of *Saccharomyces cerevisiae.*

9. Method according to Claim 8, **characterized in that** the yeast used is a strain of *Saccharomyces cerevisiae* isolated from a natural leaven.

10. Method according to Claim 9, **characterized in that** the yeast used is the *Saccharomyces cerevisiae steineri* DSM 9211 strain.

11. Use of the product obtained according to one of Claims 1 to 6, without previous separation between the culture medium and biomass, as ferment for breadmaking.

12. Use of the mother liquors resulting from the concentration according to Claim 7 for the manufacture of a substrate for the culture of lactic acid bacteria.

13. Use of the mother liquors resulting from the concentration according to claim 7 for the manufature of a drink.
